# EUROPEAN PATENT SPECIFICATION

(11) **EP 3 758 668 B1**
(45) Date of publication and mention of the grant of the patent: **23.02.2022**
(21) Application number: 19740583.0
(22) Date of filing: 22.07.2019
(51) Int. Cl.: A61K 8/11, A61K 8/88, A61K 8/06, B01J 13/16, C11D 3/50, A61Q 13/00, A01N 25/28, A23L 27/00, A23P 10/30, C11D 17/00

(54) **PROCESS FOR PREPARING MICROCAPSULES**
VERFAHREN ZUR HERSTELLUNG VON MIKROKAPSELN
PROCÉDÉ DE PRÉPARATION DE MICROCAPSULES

(30) Priority: 25.07.2018 EP 18185415
(43) Date of publication of application: 06.01.2021
(73) Proprietor: Firmenich SA, 1242 Satigny (CH)
(72) Inventor: OUALI, Lahoussine, 1242 Satigny (CH); BASSET, Jean-François, 1242 Satigny (CH); ETCHENAUSIA, Laura, 1242 Satigny (CH)
(74) Representative: Dureisseix, Valérie
(86) International application number: PCT/EP2019/069690
(87) International publication number: WO 2020/020829

(56) References cited:
- WO-A1-2013/000587
- WO-A1-2013/068255
- WO-A1-2013/092958

## Description

### TECHNICAL FIELD

The invention is strictly limited by the definition provided in the appended claims.

The present invention relates to a new process for the preparation of core-shell microcapsules. Microcapsules are also an object of the invention. Perfuming compositions and consumer products comprising said capsules, in particular perfumed consumer products in the form of home care or personal care products, are also part of the invention.

### BACKGROUND OF THE INVENTION

One of the problems faced by the perfumery industry lies in the relatively rapid loss of olfactive benefit provided by odoriferous compounds due to their volatility, particularly that of "top-notes". In order to tailor the release rates of volatiles, delivery systems such as microcapsules containing a perfume are needed to protect and later release the core payload when triggered. A key requirement from the industry regarding these systems is to survive suspension in challenging bases without physically dissociating or degrading. This is referred to as *stability* for the delivery system. For instance, fragranced personal and household cleansers containing high levels of aggressive surfactant detergents are very challenging for the stability of microcapsules.

Polyurea and polyurethane-based microcapsule slurry are widely used for example in perfumery industry as they provide a long lasting pleasant olfactory effect after their applications on different substrates. Those microcapsules have been widely disclosed in the prior art (see for example WO2007/004166 or EP 2300146 from the Applicant). See also WO2013/068255 A1, WO2013/000587 A1 and WO2013/092958 A1.

Although microcapsules are known form the prior art, there is still a need to provide new microcapsules while not compromising on their performance, in particular in terms of stability in a consumer product, as well as in delivering a good performance in terms of hydrophobic material delivery.

The present invention is proposing a solution to the above-mentioned problem, based on a new process for the preparation of microcapsules.

### SUMMARY OF THE INVENTION

It has now been found that stable core-shell microcapsules encapsulating at least one hydrophobic material could be obtained by reacting a polyfunctional 1,1-disubstituted alkene monomer with a reactant during the process, preferably during the interfacial polymerization to form microcapsules in the form of a slurry.

In a first aspect, the present invention relates to a process for the preparation of a core-shell microcapsule slurry comprising the steps of:
a) admixing at least one hydrophobic material with at least one polyfunctional 1,1-disubstituted alkene monomer to form an oil phase;
b) adding the oil phase into a water phase comprising a colloidal stabilizer to form an oil-in-water emulsion;
c) applying sufficient conditions to induce interfacial polymerization of the polyfunctional 1,1-disubstituted alkene monomer so as to form microcapsules in the form of a slurry,
wherein a reactant is added in step a) and/or in step b) and/or in step c).

In a second aspect, the invention relates to a core-shell microcapsule slurry obtainable by the process as defined above.

In a third aspect, the invention relates to a core-shell microcapsule slurry comprising at least one microcapsule made of:
- a liquid core, preferably an oil based core, and
- a shell comprising at least one polymerized polyfunctional 1,1-disubstituted alkene monomer.

A composition comprising:
(i) microcapsules as defined above, wherein the oil comprises at least one hydrophobic material;
(ii) at least one ingredient selected from the group consisting of a carrier and optionally a co-ingredient; and
(iii) optionally at least one adjuvant is another object of this invention.

Consumer products comprising:
- an active base; and
- microcapsules or a composition as defined above,
wherein the consumer products are in the form of a personal care composition or a home care composition respectively, are also part of the invention.

### FIGURE

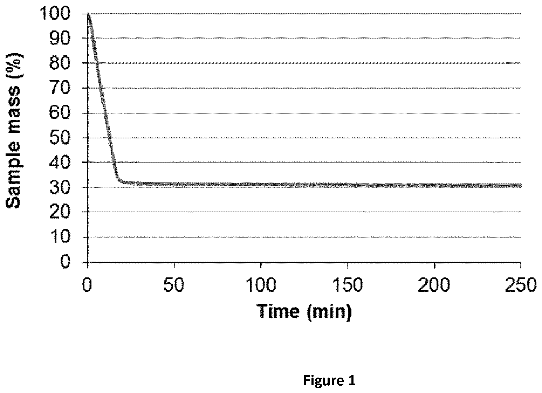

Figure 1 is a TGA profile at 50°C of invention's microcapsules.

### DETAILED DESCRIPTION OF THE INVENTION

By "hydrophobic active ingredient", it is meant a single compound or a combination of ingredients.

By "perfume or flavour oil", it is meant a single perfuming or flavouring compound or a mixture of several perfuming or flavouring compounds.

By "consumer product" or "end-product" it is meant a manufactured product ready to be distributed, sold and used by a consumer.

For the sake of clarity, by the expression "dispersion" in the present invention it is meant a system in which particles are dispersed in a continuous phase of a different composition and it specifically includes a suspension or an emulsion.

By "polyfunctional monomer", it is meant a molecule that, as unit, reacts or binds chemically to form a polymer or supramolecular polymer. The polyfunctional monomer of the invention has at least two functions capable of forming a microcapsule shell. The wording "shell" and "wall" are used indifferently in the present invention.

It has been found that stable core-shell microcapsules could be obtained when at least one polyfunctional 1,1-disubstituted alkene monomer is used during the interfacial polymerization.

### Process for preparing a microcapsule slurry

In a first aspect, the present invention relates to a process for the preparation of a core-shell microcapsule slurry comprising the steps of:
a) admixing at least one hydrophobic material with at least one polyfunctional 1,1-disubstituted alkene monomer to form an oil phase;
b) adding the oil phase into a water phase comprising a colloidal stabilizer to form an oil-in-water emulsion;
c) applying sufficient conditions to induce interfacial polymerization of the polyfunctional 1,1-disubstituted alkene monomer so as to form microcapsules in the form of a slurry,
wherein a reactant is added in step a) and/or in step b) and/or in step c).

According to an embodiment, the process for the preparation of a core-shell microcapsule slurry comprises the steps of:
a) admixing at least one hydrophobic material with at least one polyfunctional 1,1-disubstituted alkene monomer to form an oil phase;
b) adding the oil phase into a water phase comprising a colloidal stabilizer to form an oil-in-water emulsion;
c) adding to the oil-in-water emulsion of step b) a reactant to induce interfacial polymerization of the polyfunctional 1,1-disubstituted alkene monomer so as to form microcapsules in the form of a slurry.

In one step of the process, an oil phase is formed by admixing at least one hydrophobic material with at least one polyfunctional 1,1-disubstituted alkene monomer.

### Polyfunctional monomer of 1,1-disubstituted alkene compound

According to an embodiment, the polyfunctional 1,1-disubstituted alkene monomer comprises at least one 1,1-disubstituted alkene chosen in the group consisting of methylene malonates, methylene β-ketoesters, methylene β-diketones, dialkyl disubstituted vinyls, dihaloalkyl disustituted vinyls, and mixtures thereof.

According to another embodiment, the polyfunctional 1,1-disubstituted alkene monomer comprises at least two 1,1-disubstituted alkenes covalently bonded chosen in the group consisting of methylene malonates, methylene β-ketoesters, methylene β-diketones, dialkyl disubstituted vinyls, dihaloalkyl disustituted vinyls, and mixtures thereof.

According to another embodiment, the polyfunctional 1,1-disubstituted alkene monomer comprises at least three 1,1-disubstituted alkenes covalently bonded chosen in the group consisting of methylene malonates, methylene β-ketoesters, methylene β-diketones, dialkyl disubstituted vinyls, and mixtures thereof.

When the polyfunctional 1,1-disubstituted alkene monomer comprises at least two 1,1-disubstituted alkenes, they can be of the same nature or different.

According to a particular embodiment, the polyfunctional 1,1-disubstituted alkene monomer comprises at least three methylene malonates covalently bonded.

According to a particular embodiment, the polyfunctional monomer is chosen in the group consisting of O',O'-(2-(((2-(ethoxycarbonyl)acryloyl)oxy)methyl)-2-ethylpropane-1,3-diyl) 3-diethyl bis(2-methylenemalonate), O",O',O-(benzene-1,3,5-triyl) 3-triethyl tris(2-methylenemalonate), 2-ethyl-2-(((2-methylene-3-oxobutanoyl)oxy)methyl)propane-1,3-diyl bis(2-methylene-3-oxobutanoate), O',O-(1,4-phenylenebis(methylene)) 3-diethyl bis(2-methylenemalonate), benzene-1,3,5-triyltris(methylene) tris(2-methylene-3-oxobutanoate), benzene-1,3,5-triyltris(methylene) tris(2-methylene-3-oxopentanoate) and mixture thereof.

According to an embodiment, the polyfunctional 1,1-disubstituted alkene monomer comprising at least three methylene malonates is used in combination with a polyfunctional 1,1-disubstituted alkene monomer comprising two methylene malonates.

The polyfunctional monomer used in the process according to the invention is present in amounts up to 50%, preferably representing from 0.1 to 15%, more preferably from 0.5 to 10% by weight based on the total weight of the oil phase.

There is no particular restrictions regarding the way to obtain the polyfunctional 1,1-disubstituted alkene monomer that can be used in the invention.

According to an embodiment, the polyfunctional 1,1-disubstituted alkene monomer is prepared by the process comprising the steps of:
a) Preparation of an ester via esterification, and
b) Preparation of the 1,1-disubstituted alkene compounds via a Mannich reaction.

According to a particular embodiment, the polyfunctional 1,1-disubstituted alkene monomer is methylene malonate and is prepared by a process comprising the steps of:
a) Preparation of the ester malonate from acyl chloride and alcohol, and
b) Preparation of the methylene malonate *via* a Mannich reaction by reacting the ester malonate of step a) with a reactant, preferably formaldehyde or a formaldehyde precursor with ammonia or any primary or secondary amine.

The primary or secondary amine is preferably diisopropylammonium 2,2,2-trifluoroacetate.

The person skilled in the art will be able to perform a Mannich reaction based on the technical knowledge.

As used herein, "Mannich reaction" means an amino alkylation of an acidic proton placed next to a carbonyl functional group with formaldehyde and ammonia or any primary or secondary amine.

According to an embodiment, the Mannich reactions include the reaction between an diisopropylammonium 2,2,2-trifluoroacetate and O,O'-(2-(((3-ethoxy-3-oxopropanoyl)oxy)methyl)-2-ethylpropane-1,3-diyl) diethyl dimalonate, which yields a methylene malonate product.

Another object of the invention is a process for preparing a polyfunctional 1,1-disubstituted alkene monomer, said process comprising the steps of:
a. Preparation of the ester malonate from acyl chloride and alcohol
b. Preparation of the methylene malonate *via* a Mannich reaction by reacting the ester malonate of step a) with a reactant, preferably formaldehyde or a formaldehyde precursor with ammonia or any primary or secondary amine.

### Hydrophobic material

The hydrophobic material according to the invention can be "inert" material like solvents or active ingredients.

According to a particular embodiment, the hydrophobic material is a hydrophobic active ingredient.

By "hydrophobic active ingredient", it is meant any hydrophobic active ingredient - single ingredient or a mixture of ingredients - which forms a two-phase dispersion when mixed with water. The hydrophobic active ingredient is liquid at about 20°C.

Hydrophobic active ingredients are preferably chosen from the group consisting of flavor, flavor ingredients, perfume, perfume ingredients, nutraceuticals, cosmetics, pest control agents, biocide actives and mixtures thereof.

According to a particular embodiment, the hydrophobic active ingredient comprises a mixture of a perfume with another ingredient selected from the group consisting of nutraceuticals, cosmetics, pest control agents and biocide actives.

According to a particular embodiment, the hydrophobic active ingredient comprises a mixture of biocide actives with another ingredient selected from the group consisting of perfume, nutraceuticals, cosmetics, pest control agents.

According to a particular embodiment, the hydrophobic active ingredient comprises a mixture of pest control agents with another ingredient selected from the group consisting of perfume, nutraceuticals, cosmetics, biocide actives.

According to a particular embodiment, the hydrophobic active ingredient comprises a perfume.

According to a particular embodiment, the hydrophobic active ingredient consists of a perfume.

According to a particular embodiment, the hydrophobic active ingredient consists of biocide actives.

According to a particular embodiment, the hydrophobic active ingredient consists of pest control agents.

By "perfume" (or also "perfume oil") what is meant here is an ingredient or composition that is a liquid at about 20°C. According to any one of the above embodiments said perfume oil can be a perfuming ingredient alone or a mixture of ingredients in the form of a perfuming composition. As a "perfuming ingredient" it is meant here a compound, which is used for the primary purpose of conferring or modulating an odour. In other words such an ingredient, to be considered as being a perfuming one, must be recognized by a person skilled in the art as being able to at least impart or modify in a positive or pleasant way the odor of a composition, and not just as having an odor. For the purpose of the present invention, perfume oil also includes combination of perfuming ingredients with substances which together improve, enhance or modify the delivery of the perfuming ingredients, such as perfume precursors, emulsions or dispersions, as well as combinations which impart an additional benefit beyond that of modifying or imparting an odor, such as long-lasting, blooming, malodour counteraction, antimicrobial effect, microbial stability, pest control.

The nature and type of the perfuming ingredients present in the oil phase do not warrant a more detailed description here, which in any case would not be exhaustive, the skilled person being able to select them on the basis of its general knowledge and according to intended use or application and the desired organoleptic effect. In general terms, these perfuming ingredients belong to chemical classes as varied as alcohols, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said ingredients may also be compounds known to release in a controlled manner various types of perfuming compounds.

The perfuming ingredients may be dissolved in a solvent of current use in the perfume industry. The solvent is preferably not an alcohol. Examples of such solvents are diethyl phthalate, isopropyl myristate, Abalyn^{®} (rosin resins, available from Eastman), benzyl benzoate, ethyl citrate, limonene or other terpenes, or isoparaffins. Preferably, the solvent is very hydrophobic and highly sterically hindered, like for example Abalyn^{®} or benzyl benzoate. Preferably the perfume comprises less than 30% of solvent. More preferably the perfume comprises less than 20% and even more preferably less than 10% of solvent, all these percentages being defined by weight relative to the total weight of the perfume. Most preferably, the perfume is essentially free of solvent.

The term "biocide" refers to a chemical substance capable of killing living organisms (e.g. microorganisms) or reducing or preventing their growth and/or accumulation. Biocides are commonly used in medicine, agriculture, forestry, and in industry where they prevent the fouling of, for example, water, agricultural products including seed, and oil pipelines. A biocide can be a pesticide, including a fungicide, herbicide, insecticide, algicide, molluscicide, miticide and rodenticide; and/or an antimicrobial such as a germicide, antibiotic, antibacterial, antiviral, antifungal, antiprotozoal and/or antiparasite.

As used herein, a "pest control agent" indicates a substance that serves to repel or attract pests, to decrease, inhibit or promote their growth, development or their activity. Pests refer to any living organism, whether animal, plant or fungus, which is invasive or troublesome to plants or animals, pests include insects notably arthropods, mites, spiders, fungi, weeds, bacteria and other microorganisms.

According to a particular embodiment, the hydrophobic material is free of any active ingredient (such as perfume). According to this particular embodiment, it comprises solvents which can form a two-phase dispersion when mixed with water, preferably chosen in the group consisting of isopropyl myristate, tryglycerides (e.g. Neobee^{®} MCT oil, vegetable oils), D-limonene, silicone oil, mineral oil, ethyl acetate and mixtures thereof that can be used with optionally others solvents preferably chosen in the group consisting of 1,4-butanediol, benzyl alcohol, triethyl citrate, triacetin, benzyl acetate, propylene glycol (1,2-propanediol), 1,3-propanediol, dipropylene glycol, glycerol, glycol ethers and mixtures thereof.

According to any one of the invention's embodiments, the hydrophobic material represents between about 10% and 60% w/w, or even between 20% and 45% w/w, by weight, relative to the total weight of the dispersion as obtained after step b).

According to a particular embodiment, the oil phase essentially consists of polyfunctional monomer of 1,1-disubstituted alkene compound and at least one hydrophobic material.

In another step of the process according to the invention, the oil phase of step a) is dispersed into an aqueous solution comprising a colloidal stabilizer to form an oil-in-water emulsion.

### Colloidal stabilizer

The colloidal stabilizer used in the present invention can be a molecular colloidal stabilizer or solid particles.

According to an embodiment, the colloidal stabilizer is an ionic colloidal stabilizer chosen in the group consisting of gum Arabic, carboxymethyl cellulose, soy protein, sodium caseinate, gelatin, bovine serum albumin, sugar beet pectin, hydrolyzed soy protein, hydrolyzed sericin, Pseudocollagen, Biopolymer SA-N ((INCI Name: Hyaluronic Acid (and) Albumen (and) Dextran Sulfate; origin Lipo Chemicals), Pentacare-NA PF (INCI Name: Hydrolyzed Wheat Gluten (and) Ceratonia Siliqua (Carob) Gum (and) Aqua (and) Sodium Dextran Sulfate (and) Bis-Hydroxyethyl Tromethamine (and) Phenoxyethanol (and) Ethylhexylglycerin; origin DSM Nutritional Products, LLC), lignin derivatives such as lignosulfonic acid salts and mixtures thereof.

According to another embodiment, the colloidal stabilizer is a non-ionic emulsifier chosen in the group consisting of polyvinyl alcohol, modified polyvinyl alcohol, modified starch, modified cellulose, polysaccharides, lignin, and mixtures thereof.

According to a preferred embodiment the colloidal stabilizer is chosen in the group consisting of gum Arabic, modified starch, polyvinyl alcohol, polyvinylpyrrolidone (PVP), carboxymethylcellulose (CMC), anionic polysaccharides, acrylamide copolymer, lignin and derivatives, inorganic particles and mixtures thereof.

According to any one of the above embodiments of the present invention, the emulsion comprises between about 0.1% and 5% w/w of at least a colloid stabilizer, percentage being expressed on a w/w basis relative to the total weight of the emulsion as obtained after step b). In still another aspect of the invention, the emulsion comprises between about 0.1% and 2% w/w of at least a colloid stabilizer.

The emulsion may be prepared by high shear mixing and adjusted to the desired droplet size. The mean droplet size of the emulsion is preferably comprised between 1 and 1000 microns, more preferably between 1 and 500 microns, and even more preferably between 5 and 50 microns. The droplet size can be checked with light scattering measurements or microscopy. This procedure does not require a more detailed description here as it is well known to a skilled person in the art.

According to the invention, a reactant is added in step a) and/or in step b) and/or in step c).

According to a particular embodiment, in step c) of the process, a reactant is added to the oil-in-water emulsion of step b) to induce interfacial polymerization of the polyfunctional 1,1-disubstituted alkene monomer so as to form microcapsules in form of a slurry.

### Reactant

Among the reactant that can be used in the invention, one may cite nucleophile compounds and/or free radical initiator (*i*.*e*., polymerization activator).

According to the invention, a nucleophile compound is defined as a chemical species that donates an electron pair to an electrophile to form a chemical bond in relation to a reaction.

The nucleophile compound may be chosen in the group consisting of nitrogen nucleophile, sulfur nucleophile, oxygen nucleophile, carbon nucleophile, phosphor nucleophile, and mixtures thereof.

Nitrogen nucleophile compound may have at least one functional group chosen in the group consisting of ammonia, azide, amines, nitrites, hydroxylamine, hydrazin, carbazide, phenylhydrazine, semicarbazide, and amide, and mixtures thereof.

Sulfur nucleophile compound may have at least one functional group chosen in the group consisting of hydrogen sulfide and its salts, thiols (RSH), thiolate anions (RS-), anions of thiolcarboxylic acids (RC(O)-S-), and anions of dithiocarbonates (RO-C(S)-S-) and dithiocarbamates (R2N-C(S)-S-) and mixtures therof.

Oxygen nucleophile compound may have at least one functional group chosen in the group consisting of water, hydroxide anion, alcohols, alkoxide anions, carboxylate anions, carbonates, sulfonate, sulfate, sodium phosphates, sodium silicates, borax, sodium tetraborate, and mixtures thereof.

Carbone nucleophile compound may have at least one functional group chosen in the group consisting of enols carbon nucleophiles, malonate and acetoacetate.

Phosphore nucleophile compound may have at least one functional group chosen in the group consisting of phosphine, phosphite anion and mixture thereof.

According to a particular embodiment, the reactant is chosen in the group consisting of xylylene diamine, 1,2-diaminocyclohexane, 1,4-diaminocyclohexane, L-lysine, L-Lysine ethyl ester, Jeffamine^{®} (O,O'-Bis(2-aminopropyl) polypropylene glycol-block-polyethylene glycol-block-polypropylene glycol), ethylene diamine, 1,3-diamino-2-hydroxypropane, diethylene triamine, spermine, spermidine, cystamine, cystine, cystine dialkyl ester, aminoguanidine bicarbonate, N,N'-diethylethylenediamine, polyamidoamine (PAMAM), chitosan, 3-aminopropyltriethoxysilane, L-arginine, 1,3-diaminopropane, N-ethylguanidine sulfate, 1,6-diaminohexane, guanidine salts, N,N,N',N'-tetrakis(3-aminopropyl)-1,4-butanediamine, guanazole, 2-amino-1,3-propanediol, ethanolamine, tris(2-aminoethyl)amine, Tris(3-aminopropyl)amine, Tris[2-(methylamino)ethyl]amine, 1-(2-Aminoethyl)piperazine, Triethylenetetramine, triethanolamine, phthalic acid dipotassium salt, succinic acid disodium salt, dithiothreitol, trimethylolpropane tris(3-mercaptopropionate), pentaerythritol tetrakis(3-mercaptopropionate), 2,2'-thiodiethanethiol and mixtures thereof.

The most common classes of free radical initiators that can be used in the present invention are peroxides, azo compounds and persulfates. Radicals may be generated by thermal or ambient redox conditions.

The wall of the microcapsules is the result of the interfacial polymerization between the polyfunctional 1,1-disubstituted alkene monomer and the reactant, preferably added in step c).

The amount of reactant used is typically adjusted so that the molar ratio between the reactive groups of the reactant and the reactive groups of the 1,1-disubstituted alkene monomer is comprised from 0.1 to 10, preferably between 1 and 5.

By "reactive groups of the reactant", it is meant nucleophile groups of the reactant as defined previously.

By "reactive groups of the 1,1-disubstituted alkene monomer", it is meant methylene group.

No specific action is required to induce the interfacial polymerization of the polyfunctional 1,1-disubstituted alkene monomer. Preferably the reaction is maintained for 2 to 15 hours, more preferably for 2 to 10 hours. To increase the kinetic reaction, said step may be performed at a temperature comprised between 60 and 80°C, possibly under pressure, for 1 to 4 hours. More preferably it is performed at between 50 and 90°C for between 30 minutes and 4 hours.

According to a particular embodiment of the invention, at the end of step c) or during step c), one may also add to the invention's slurry a polymer selected from cationic polymer, a polysaccharide and mixtures thereof to form an outer coating to the microcapsules.

Polysaccharide polymers are well known to a person skilled in the art. Preferred non-ionic polysaccharides are selected from the group consisting of locust bean gum, xyloglucan, guar gum, hydroxypropyl guar, hydroxypropyl cellulose and hydroxypropyl methyl cellulose, pectin and mixtures thereof.

Cationic polymers are well known to a person skilled in the art. Preferred cationic polymers have cationic charge densities of at least 0.5 meq/g, more preferably at least about 1.5 meq/g, but also preferably less than about 7 meq/g, more preferably less than about 6.2 meq/g. The cationic charge density of the cationic polymers may be determined by the Kjeldahl method as described in the US Pharmacopoeia under chemical tests for Nitrogen determination. The preferred cationic polymers are chosen from those that contain units comprising primary, secondary, tertiary and/or quaternary amine groups that can either form part of the main polymer chain or can be borne by a side substituent directly connected thereto. The weight average (Mw) molecular weight of the cationic polymer is preferably between 10,000 and 3.5M Dalton, more preferably between 50,000 and 1.5M Dalton. According to a particular embodiment, one will use cationic polymers based on acrylamide, methacrylamide, N-vinylpyrrolidone, quaternized N,N-dimethylaminomethacrylate, diallyldimethylammonium chloride, quaternized vinylimidazole (3-methyl-1-viny1-1H-imidazol-3-ium chloride), vinylpyrrolidone, acrylamidopropyltrimonium chloride, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride. Preferably copolymers shall be selected from the group consisting of polyquaternium-5, polyquaternium-6, polyquaternium-7, polyquatemium10, polyquaternium-11, polyquaternium-16, polyquaternium-22, polyquaternium-28, polyquaternium-43, polyquaternium-44, polyquaternium-46, cassia hydroxypropyltrimonium chloride, guar hydroxypropyltrimonium chloride or polygalactomannan 2-hydroxypropyltrimethylammonium chloride ether, starch hydroxypropyltrimonium chloride and cellulose hydroxypropyltrimonium chloride. As specific examples of commercially available products, one may cite Salcare^{®} SC60 (cationic copolymer of acrylamidopropyltrimonium chloride and acrylamide, origin: BASF) or Luviquat^{®}, such as the PQ 11N, FC 550 or Style (polyquaternium-11 to 68 or quaternized copolymers of vinylpyrrolidone origin: BASF), or also the Jaguar^{®} (C13S or C17, origin Rhodia).

According to any one of the above embodiments of the invention, there is added an amount of polymer described above comprised between about 0% and 5% w/w, or even between about 0.1% and 2% w/w, percentage being expressed on a w/w basis relative to the total weight of the slurry as obtained after step c). It is clearly understood by a person skilled in the art that only part of said added polymers will be incorporated into/deposited on the microcapsule shell.

### Process for preparing a microcapsule powder

Another object of the invention is a process for preparing a microcapsule slurry comprising the steps as defined above and an additional step d) consisting of submitting the slurry obtained in step c) to a drying, like spray-drying, to provide the microcapsules as such, i.e. in a powdery form. It is understood that any standard method known by a person skilled in the art to perform such drying is also applicable. In particular the slurry may be spray-dried preferably in the presence of a polymeric carrier material such as polyvinyl acetate, polyvinyl alcohol, dextrins, natural or modified starch, vegetable gums, pectins, xanthans, alginates, carragenans or cellulose derivatives to provide microcapsules in a powder form.

However, one may cite also other drying method such as the extrusion, the fluidized bed, or even a drying at room temperature using materials (carrier, desiccant) that meet specific criteria as disclosed in WO2017/134179.

### Microcapsule slurry/Microcapsule powder

Another object of the invention is a core-shell microcapsule slurry comprising at least one microcapsule made of:
- a liquid core, preferably an oil based core, and
- a shell comprising at least one polymerized polyfunctional 1,1-disubstituted alkene monomer.

A microcapsule slurry obtainable by the process as described above is also an object of the invention.

Still another object of the invention is a microcapsule powder obtained by drying the microcapsules slurry described above.

The embodiments described previously for the process for preparing microcapsules also apply for the microcapsule slurry/powder described previously.

### Perfuming composition/consumer products

Another object of the invention is a composition comprising
(i) microcapsules as defined above, wherein the oil comprises a hydrophobic material;
(ii) at least one ingredient selected from the group consisting of a carrier and optionally a co-ingredient; and
(iii) optionally at least one adjuvant is another object of this invention.

Another object of the present invention is a perfuming composition comprising:
(i) microcapsules as defined above, wherein the oil-based core comprises a perfume;
(ii) at least one ingredient selected from the group consisting of a perfumery carrier, a perfumery co-ingredient and mixtures thereof;
(iii) optionally at least one perfumery adjuvant.

As liquid perfumery carrier one may cite, as non-limiting examples, an emulsifying system, i.e. a solvent and a surfactant system, or a solvent commonly used in perfumery. A detailed description of the nature and type of solvents commonly used in perfumery cannot be exhaustive. However, one can cite as non-limiting examples solvents such as dipropyleneglycol, diethyl phthalate, isopropyl myristate, benzyl benzoate, 2-(2-ethoxyethoxy)-1-ethanol or ethyl citrate, which are the most commonly used. For the compositions which comprise both a perfumery carrier and a perfumery co-ingredient, other suitable perfumery carriers than those previously specified, can be also ethanol, water/ethanol mixtures, limonene or other terpenes, isoparaffins such as those known under the trademark Isopar^{®} (origin: Exxon Chemical) or glycol ethers and glycol ether esters such as those known under the trademark Dowanol^{®} (origin: Dow Chemical Company). By "perfumery co-ingredient" it is meant here a compound, which is used in a perfuming preparation or a composition to impart a hedonic effect and which is not a microcapsule as defined above. In other words such a co-ingredient, to be considered as being a perfuming one, must be recognized by a person skilled in the art as being able to at least impart or modify in a positive or pleasant way the odor of a composition, and not just as having an odor.

The nature and type of the perfuming co-ingredients present in the perfuming composition do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

Preferably, the perfuming composition according to the invention comprises between 0.1 and 30 % by weight of microcapsules as defined above.

The microcapsules of the invention can also be added in different perfumed consumer products.

In particular a perfuming composition comprising (i) microcapsules as defined above; (ii) at least one perfuming co-ingredient; and (iii) optionally a perfumery adjuvant, is another object of the invention.

By "perfuming co-ingredient" it is meant here a compound, which is used in a perfuming preparation or a composition to impart a hedonic effect and which is not a microcapsule as defined above. In other words such a co-ingredient, to be considered as being a perfuming one, must be recognized by a person skilled in the art as being able to impart or modify in a positive or pleasant way the odor of a composition, and not just as having an odor. The nature and type of the perfuming co-ingredients present in the perfuming composition do not warrant a more detailed description here, which in any case would not be exhaustive, the skilled person being able to select them on the basis of his general knowledge and according to the intended use or application and the desired organoleptic effect. In general terms, these perfuming co-ingredients belong to chemical classes as varied as alcohols, lactones, aldehydes, ketones, esters, ethers, acetates, nitriles, terpenoids, nitrogenous or sulphurous heterocyclic compounds and essential oils, and said perfuming co-ingredients can be of natural or synthetic origin. Many of these co-ingredients are in any case listed in reference texts such as the book by S. Arctander, Perfume and Flavor Chemicals, 1969, Montclair, New Jersey, USA, or its more recent versions, or in other works of a similar nature, as well as in the abundant patent literature in the field of perfumery. It is also understood that said co-ingredients may also be compounds known to release in a controlled manner various types of perfuming compounds.

By "perfumery adjuvant" we mean here an ingredient capable of imparting additional added benefit such as a color, a particular light resistance, chemical stability, etc. A detailed description of the nature and type of adjuvant commonly used in perfuming bases cannot be exhaustive, but it has to be mentioned that said ingredients are well known to a person skilled in the art.

Preferably, the perfuming composition according to the invention comprises between 0.1 and 30 % by weight of microcapsules as defined above.

The invention's microcapsules can advantageously be used in many application fields and used in consumer products. Microcapsules can be used in liquid form applicable to liquid consumer products as well as in powder form, applicable to powder consumer products.

In the case of microcapsules including a perfume oil-based core, the products of the invention, can in particular be of used in perfumed consumer products such as product belonging to fine fragrance or "functional" perfumery. Functional perfumery includes in particular personal-care products including hair-care, body cleansing, skin care, hygiene-care as well as home-care products including laundry care and air care. Consequently, another object of the present invention consists of a perfumed consumer product comprising as a perfuming ingredient, the microcapsules defined above or a perfuming composition as defined above. The perfume element of said consumer product can be a combination of perfume microcapsules as defined above and free or non-encapsulated perfume, as well as other types of perfume microcapsule than those here-disclosed.

In particular a liquid consumer product comprising:
- from 2 to 65% by weight, relative to the total weight of the consumer product, of at least one surfactant;
- water or a water-miscible hydrophilic organic solvent; and
- a perfuming composition or microcapsules as defined above, wherein the hydrophobic active ingredient comprise a perfume is another object of the invention.

Also a powder consumer product comprising
- from 2 to 65% by weight, relative to the total weight of the consumer product, of at least one surfactant; and
- a perfuming composition or a microcapsule powder, wherein the hydrophobic active ingredient comprise a perfume as defined above is part of the invention.

The invention's microcapsules can therefore be added as such or as part of an invention's perfuming composition in a perfumed consumer product.

For the sake of clarity, it has to be mentioned that, by "perfumed consumer product" it is meant a consumer product which is expected to deliver among different benefits a perfuming effect to the surface to which it is applied (e.g. skin, hair, textile, paper, or home surface) or in the air (air-freshener, deodorizer etc). In other words, a perfumed consumer product according to the invention is a manufactured product which comprises a functional formulation also referred to as "base", together with benefit agents, among which an effective amount of microcapsules according to the invention.

The nature and type of the other constituents of the perfumed consumer product do not warrant a more detailed description here, which in any case would not be exhaustive, the skilled person being able to select them on the basis of his general knowledge and according to the nature and the desired effect of said product. Base formulations of consumer products in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

Non-limiting examples of suitable perfumed consumer product can be a perfume, such as a fine perfume, a cologne, an after-shave lotion, a body-splash; a fabric care product, such as a liquid or solid detergent, tablets and pods, a fabric softener, a dryer sheet, a fabric refresher, an ironing water, or a bleach; a personal-care product, such as a hair-care product (e.g. a shampoo, hair conditioner, a colouring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product); an air care product, such as an air freshener or a "ready to use" powdered air freshener; or a home care product, such all-purpose cleaners, liquid or power or tablet dishwashing products, toilet cleaners or products for cleaning various surfaces, for example sprays & wipes intended for the treatment / refreshment of textiles or hard surfaces (floors, tiles, stone-floors etc.); a hygiene product such as sanitary napkins, diapers, toilet paper.

Another object of the invention is a consumer product comprising:
a personal care active base, and
microcapsules as defined above or the perfuming composition as defined above,
wherein the consumer product is in the form of a personal care composition.

Personal care active base in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature.

The personal care composition is preferably chosen in the group consisting of a hair-care product (e.g. a shampoo, hair conditioner, a colouring preparation or a hair spray), a cosmetic preparation (e.g. a vanishing cream, body lotion or a deodorant or antiperspirant), or a skin-care product (e.g. a perfumed soap, shower or bath mousse, body wash, oil or gel, bath salts, or a hygiene product).

Another object of the invention is a consumer product comprising:
a home care or a fabric care active base, and
microcapsules as defined above or the perfuming composition as defined above,
wherein the consumer product is in the form of a home care or a fabric care composition.

Home care or fabric care bases in which the microcapsules of the invention can be incorporated can be found in the abundant literature relative to such products. These formulations do not warrant a detailed description here which would in any case not be exhaustive. The person skilled in the art of formulating such consumer products is perfectly able to select the suitable components on the basis of his general knowledge and of the available literature. The home or fabric care composition is preferably chosen in the group consisting fabric softener, liquid detergent, powder detergent, liquid scent booster solid scent booster.

According to a particular embodiment, the consumer product is in the form of a fabric softener composition and comprises:
- between 85 and 99.9% of a fabric softener active base;
- between 0.1 to 15 wt%, more preferably between 0.2 and 5 wt% by weight of the microcapsule slurry of the invention.

The fabric softener active base may comprise cationic surfactants of quaternary ammonium, such as Dialkyl ester dimethyl ammonium chloride (DEEDMAC), TEAQ (triethanolamine quat), HEQ (Hamburg esterquat), and mixtures thereof.

Preferably, the consumer product comprises from 0.1 to 15 wt%, more preferably between 0.2 and 5 wt% of the microcapsules of the present invention, these percentages being defined by weight relative to the total weight of the consumer product. Of course the above concentrations may be adapted according to the benefit effect desired in each product.

The invention will now be further described by way of examples. It will be appreciated that the invention as claimed is not intended to be limited in any way by these examples.

### Examples

### Example 1

### O',O'-(2-(((2-(ethoxycarbonyl)acryloyl)oxy)methyl)-2-ethylpropane-1,3-diyl) 3-diethyl bis(2-methylenemalonate) monomer synthesis (TriDEMM)

### I- Preparation of O,O'-(2-(((3-ethoxy-3-oxopropanoyl)oxy)methyl)-2-ethylpropane-1,3-diyl) diethyl dimalonate

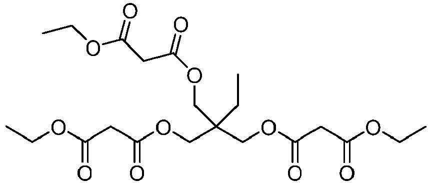

2-ethyl-2-(hydroxymethyl)propane-1,3-diol (4.45 g, 33.2 mmol), ethyl malonyl monochloride (21.23 ml, 166 mmol), and pyridine (13.41 ml, 166 mmol) were mixed in dichloromethane (491 ml, 7628 mmol)/ tetrahydrofuran (82 ml, 995 mmol) and the solution was stirred for 12 h at 20°C.

The mixture was washed with saturated aqueous NH₄Cl solution (2 × ), dried (Na₂SO₄), and evaporated to dryness. Column chromatography (SiO₂;CH₂Cl₂/AcOEt 3:1) yielded the desired product O,O'-(2-(((3-ethoxy-3-oxopropanoyl)oxy)methyl)-2-ethylpropane-1,3-diyl) diethyl dimalonate (15.5 g, 31.9 mmol, 96 % yield) as a colorless oil.

### II- Preparation of O',O'-(2-(((2-(ethoxycarbonyl)acryloyl)oxy)methyl)-2-ethylpropane-1,3-diyl) 3-diethyl bis(2-methylenemalonate) monomer synthesis (TriDEMM)

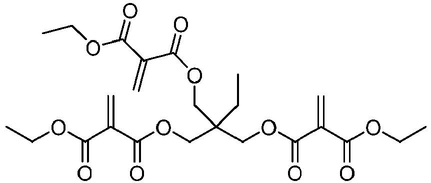

Dry THF (91 ml, 1109 mmol), O,O'-(2-(((3-ethoxy-3-oxopropanoyl)oxy)methyl)-2-ethylpropane-1,3-diyl) diethyl dimalonate (15 g, 31.5 mmol), diisopropylammonium 2,2,2-trifluoroacetate (20.33 g, 94 mmol), paraformaldehyde (5.67 g, 189 mmol) and 2,2,2-trifluoroacetic acid (0.723 ml, 9.44 mmol) were added to a 250 mL round bottom flask. A condenser was added and the suspension was stirred to reflux for two hours. Paraformaldehyde (5.67 g, 189 mmol) was added and the reflux was restarted for 6 hours. The reaction was cooled to room temperature and the oil for tests (support for polymerization) was added (38.90g) and THF was removed under reduced pressure (300 to 50 mbar at 45°C). The crude mixture was dissolved in diethyl ether (250 mL) and filtered through cotton in a separatory funnel. The organic layer was washed twice with 1 M HCl (100 mL). The aqueous layers were combined and extracted with diethyl ether (50 mL). The organic layers were combined, dried with anhydrous Na₂SO₄, filtered and concentrated under reduced pressure to give a yellow oil (70 g).

### Example 2

### O",O',O-(benzene-1,3,5-triyl) 3-triethyl tris(2-methylenemalonate) monomer synthesis (Aromatic TriDEMM)

### I- Preparation of O,O',O"-benzene-1,3,5-triyl triethyl trimalonate

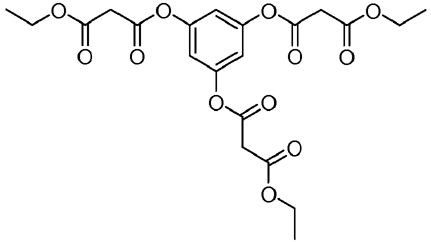

Benzene-1,3,5-triol (2 g, 15.86 mmol), ethyl malonyl monochloride (10.15 ml, 79 mmol), and pyridine (6.41 ml, 79 mmol) were mixed in dichloromethane (235 ml, 3648 mmol)/ THF (39.0 ml, 476 mmol) and the solution was stirred for 12 h at 20°C. The mixture was filtred, washed with saturated aqueous NH₄Cl solution (2 x), dried (Na₂SO₄), and evaporated to dryness. Column chromatography (SiO₂;CH₂Cl₂/AcOEt 3:1) yielded the desired product as a colorless oil.

### II- Preparation of O",O',O-(benzene-1,3,5-triyl) 3-triethyl tris(2-methylenemalonate) monomer synthesis (Aromatic TriDEMM)

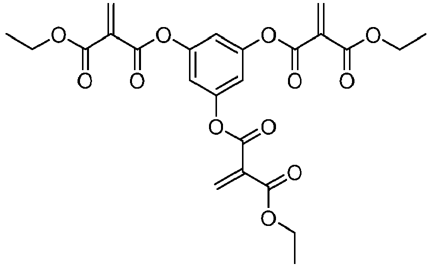

Dry THF (14.67 ml, 179 mmol), O,O',O"-benzene-1,3,5-triyl triethyl trimalonate (2.38 g, 5.08 mmol), diisopropylammonium 2,2,2-trifluoroacetate (3.28 g, 15.24 mmol), paraformaldehyde

(0.915 g, 30.5 mmol) and 2,2,2-trifluoroacetic acid (0.117 ml, 1.524 mmol) were added to a 50 mL round bottom flask. A condenser was added and the suspension was stirred to reflux for two hours. Paraformaldehyde (0.915 g, 30.5 mmol) was added and the reflux was restarted for 6 hours. The reaction was cooled to room temperature and the oil for tests (support for polymerization) was added (5.97 g) and THF was removed under reduced pressure (300 to 50 mbar at 45°C). The crude mixture was dissolved in diethyl ether (40 mL) and filtered through cotton in a separatory funnel. The organic layer was washed twice with 1 M HCl (15 mL). The aqueous layers were combined and extracted with diethyl ether (8 mL). The organic layers were combined, dried with anhydrous Na₂SO₄, filtered and concentrated under reduced pressure to give a yellow oil.

### Example 3

### 2-ethyl-2-(((2-methylene-3-oxobutanoyl)oxy)methyl)propane-1,3-diyl bis(2-methylene-3-oxobutanoate) monomer synthesis (Triacetoacetate)

### I- Preparation of 2-ethyl-2-(((3-oxobutanoyl)oxy)methyl)propane-1,3-diyl bis(3-oxobutanoate)

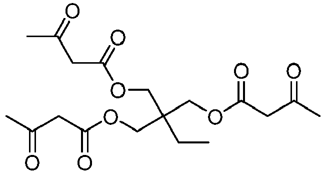

Trimethylolpropane (1.1 g, 8.20 mmol) is added to xylene (10 ml, 27.0 mmol) and the solution is heated to 125°C. 2,2,6-Trimethyl-4H-1,3-dioxin-4-one (6.13 g, 41.0 mmol) is added slowly (2.5ml/h). The mixture is kept at 125°C in an open flask.

### II- Preparation of 2-ethyl-2-(((2-methylene-3-oxobutanoyl)oxy)methyl)propane-1,3-diyl bis(2-methylene-3-oxobutanoate) (Triacetoacetate)

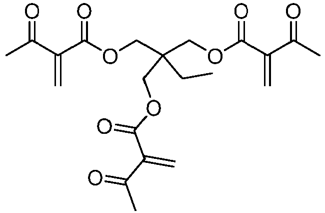

Dry THF (14.90 ml, 182 mmol) , 2-ethyl-2-(((3-oxobutanoyl)oxy)methyl)propane-1,3-diyl bis(3-oxobutanoate) (1 g, 2.59 mmol) , diisopropylammonium 2,2,2-trifluoroacetate (1.671 g, 7.76 mmol) , paraformaldehyde (0.466 g, 15.53 mmol) and 2,2,2-trifluoroacetic acid (0.059 ml, 0.776 mmol) were added to a 50 mL round bottom flask. A condenser was added and the suspension was stirred to reflux for two hours. paraformaldehyde (0.466 g, 15.53 mmol) was added and the reflux was restarted for 6 hours. The reaction was cooled to room temperature and the oil for tests (support for polymerization) was added (2.55 g) and THF was removed under reduced pressure (300 to 50 mbar at 45°C). The crude mixture was dissolved in ethyl acetate (25 mL) (not soluble in diethyl ether) and filtered through cotton in a separatory funnel. The organic layer was washed twice with 1 M HCl (15 mL). The aqueous layers were combined and extracted with ethyl acetate (8 mL). The organic layers were combined, dried with anhydrous Na₂SO₄, filtered and concentrated under reduced pressure to give a yellow oil.

### Example 4

### O,O'-(1,4-phenylenebis(methylene)) 3-diethyl bis(2-methylenemalonate) monomer synthesis (Aromatic diDEMM)

### I- Preparation of O,O'-(1,4-phenylenebis(methylene)) diethyl dimalonate

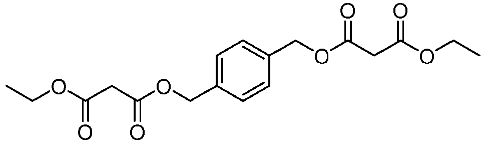

1,4-phenylenedimethanol (2g, 14.48 mmol) , ethyl malonyl monochloride (5.56 ml, 43.4 mmol) , and pyridine (3.51 ml, 43.4 mmol) were mixed in dichloromethane (214 ml, 3329 mmol) / tetrahydrofuran (35.6 ml, 434 mmol) and the solution was stirred for 12 h at 20°C. The mixture was washed with saturated aqueous NH₄Cl solution (2 × ), dried (Na₂SO₄), and evaporated to dryness. Column chromatography (SᵢO₂;CH₂Cl₂/AcOEt 3:1) yielded the desired product as a colorless oil.

### II- Preparation of O,O'-(1,4-phenylenebis(methylene)) 3-diethyl bis(2-methylenemalonate)

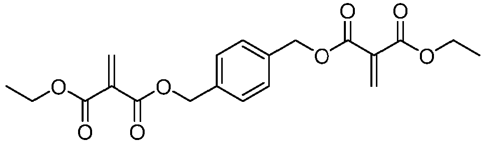

Dry THF (35.5 ml, 433 mmol) , O,O'-(1,4-phenylenebis(methylene)) diethyl dimalonate (4.5 g, 12.28 mmol) , diisopropylammonium 2,2,2-trifluoroacetate (5.29 g, 24.57 mmol) , paraformaldehyde (1.475 g, 49.1 mmol) and 2,2,2-trifluoroacetic acid (0.188 ml, 2.457 mmol) were added to a 100 mL round bottom flask. A condenser was added and the suspension was stirred to reflux for two hours. Paraformaldehyde (1.475 g, 49.1 mmol) was added and the reflux was restarted for 6 hours. The reaction was cooled to room temperature and the oil for tests (support for polymerization) was added (11.2g) and THF was removed under reduced pressure (300 to 50 mbar at 45°C). The crude mixture was dissolved in diethyl ether (25 mL) and filtered through cotton in a separatory funnel. The organic layer was washed twice with 1 M HCl (15 mL). The aqueous layers were combined and extracted with diethyl ether (8 mL). The organic layers were combined, dried with anhydrous Na₂SO₄, filtered and concentrated under reduced pressure to give a yellow oil (16 g).

### Example 5

### Preparation of microcapsules according to the invention

Microcapsules A-G were prepared according to the following protocol.

### General protocol:

An aqueous solution of stabilizer is prepared by dissolving 2 wt% of Gum Arabic Superstab AA (origin: Nexira, France) in deionized water. In a separate container, the oil phase is formulated by dissolving monomers (as prepared in example 1 (TriDEMM), 2 (aromatic TriDEMM), 3 (triacetoacetate) or 4 (aromatic diDEMM)) in a perfume oil A (see table 1) or in a solvent. The oil phase is poured into the water phase and the mixture is emulsified with an Ultra-Turrax disperser at 24 000 rpm for 30s to form the oil-in-water emulsion. The emulsion is introduced into a jacketed reactor, fitted with a reflux condenser and stainless steel stirrer, and stirred at 400 rpm. In a flask, an aqueous solution of tris(2-aminoethyl)amine (origin: Alfa Aesar, Switzerland) is prepared and added dropwise to the oil-in-water emulsion. The reaction mixture is stirred at room temperature for at least 2 hours or at 60°C for 2 hours. The final product is recovered as a milky dispersion.

**Table 1: Perfume oil A composition**

| **Raw material** | **wt%** |
|---|---|
| *Romascone*^{®*a)*} | 33.3 |
| *Verdox^{TMb)}* | 33.3 |
| *Lorysia*^{®*c)*} | 33.3 |

*a) Methyl 2,2-dimethyl-6-methylene-1-cyclohexanecarboxylate, origin: Firmenich SA, Geneva, Switzerland*
*b) 2-tert-butyl-1-cyclohexyl acetate, trademark from International Flavors* & *Fragrances, USA*
*c) 4-(1,1-dimethylethyl)-1-cyclohexyl acetate, origin: Firmenich SA, Geneva, Switzerland*

**Table 2: Microcapsule compositions (based on triDEMM - prepared according to example 1)**

| **Capsules** | **triDEMM (g)** | **triamine (g)** | **Perfume (g)** | **Gum arabic solution at 2 wt% (g)** | **[NH₂]/[double bond] (mol/mol)** | **triDEMM/perfume (g/g)** |
|---|---|---|---|---|---|---|
| A | 3.835 | 1.65 | 59.12 | 134.06 | 1.5 | 0.065 |
| B | 2.50 | 1.1 | 19.00 | 44.39 | 1.5 | 0.13 |
| C | 2.50 | 1.66 | 19.01 | 43.98 | 2.3 | 0.13 |
| D | 7.67 | 1.65 | 59.15 | 134.24 | 0.7 | 0.13 |

**Table 3: Microcapsule composition (based on aromatic triDEMM - prepared according to example 2)**

| **Capsules** | **Aromatic triDEMM (g)** | **triamine (g)** | **Ethyl acetate (g)** | **Gum arabic solution at 2 wt% (g)** | **[NH₂]/[double bond] (mol/mol)** | **Aromatic triDEMM/solvent (g/g)** |
|---|---|---|---|---|---|---|
| E | 1.3 | 0.72 | 20 | 46 | 1.5 | 0.065 |

**Table 4: Microcapsule composition (based on triacetoacetate - prepared according to example 3)**

| **Capsule s** | **triacetoacetate (g)** | **triami ne (g)** | **Ethyl acetat e (g)** | **Gum arabic solutio n at 2 wt% (g)** | **[NH₂]/[doubl e bond] (mol/mol)** | **triacetoacetate/sov ent (g/g)** |
|---|---|---|---|---|---|---|
| F | 0.36 | 0.22 | 3 | 8 | 1.8 | 0.12 |

**Table 5: Microcapsule composition (based on aromatic diDEMM - prepared according to example 4)**

| **Capsules** | **Aromatic diDEMM (g)** | **triamine (g)** | **Perfume (g)** | **Gum arabic solution at 2 wt% (g)** | **[NH₂]/[double bond] (mol/mol)** | **Aromatic diDEMM/perfume (g/g)** |
|---|---|---|---|---|---|---|
| G | 3.84 | 1.47 | 59.0 | 136.34 | 1.5 | 0.065 |

### Example 6

### Shell composition by elemental analysis

Shell were extracted and analyzed by elemental analysis. Composition was estimated by calculation based on the component compositions.

**Table 6: Composition of the extracted and purified shell of capsule A (example 5) determined by elemental analysis**

| Capsules | %C | %H | %N | %O |
|---|---|---|---|---|
| A | 53.0 | 6.5 | 3.4 | 34.6 |

The results of the elemental analysis show the presence of nitrogen in the shell of the capsules which underlines that the membrane results from the reaction of the tris(2-aminoethyl)amine and the triDEMM monomer.

### Example 7

### Capsule performance at 50°C

TGA: Capsule performance was assessed at 50°C with a thermogravimetric analyser (TGA/SDTA851e, Origin: Mettler-Toledo, Switzerland) equipped with a microbalance (accuracy: 1 µg) and an accurate oven having an internal volume of 35 ml, under a constant nitrogen flow of 20ml/min. Perfume evaporation was measured as a function of time. Microcapsules dispersion (10 mg) was introduced in alumina pan of 100 µl. The measurement at 50°C started from 25°C to 50°C at 5°C/min, and then staid at 50°C for 4 h. A slower evaporation of the perfume oil with a long-lasting profile was related to a more stable capsule.

Figure 1 shows that microcapsules A are stable.

### Example 8

### Fabric softener composition

Capsules A, B, C or D of the present invention are dispersed in a fabric softener base described in table 7 to obtain a concentration of encapsulated perfume oil at 0.22%.

**Table 7: Fabric Softener composition**

| **Product** | **Wt %** |
|---|---|
| Stepantex VL 90A | 8.88 |
| Calcium Chloride Sol. 10% | 0.36 |
| Proxel GXL | 0.04 |
| Perfume | 1 |
| Water | 89.72 |
| TOTAL | 100 |

### Example 9

### Liquid detergent composition

Capsules A, B, C or D of the present invention are dispersed in a liquid detergent base described in table 8 to obtain a concentration of encapsulated perfume oil at 0.22%.

**Table 8: Liquid detergent composition**

| Ingredients | Concentration [wt%] |
|---|---|
| Sodium C14-17 Alkyl Sec Sulfonate¹⁾ | 7 |
| Fatty acids, C12-18 and C18-unsaturated²⁾ | 7.5 |
| C12/14 fatty alcohol polyglycol ether with 7 mol EO³⁾ | 17 |
| Triethanolamine | 7.5 |
| Propylene Glycol | 11 |
| Citric acid | 6.5 |
| Potassium Hydroxyde | 9.5 |
| Protease | 0.2 |
| Amylase | 0.2 |
| Mannanase | 0.2 |
| Acrylates/Steareth-20 Methacrylate structuring Crosspolymer⁴⁾ | 6 |
| Deionized Water | 27.4 |

1) Hostapur SAS 60; Origin: Clariant
2) Edenor K 12-18; Origin: Cognis
3) Genapol LA 070; Origin: Clariant
4) Aculyn 88; Origin: Dow Chemical

### Example 10

### Rinse-off conditioner

Capsules A, B, C or D of the present invention are dispersed in a rinse-off conditioner base described in table 9 to obtain a concentration of encapsulated perfume oil at 0.5%.

**Table 9: Rinse-off conditioner composition**

| | **Ingredients** | **Concentration [wt%]** |
|---|---|---|
| **A** | Water deionized | **81.8** |
| | Behentrimonium Chloride ¹⁾ | **2.5** |
| | Hydroxyethylcellulose ²⁾ | **1.5** |
| | | |
| **B** | Cetearyl Alcohol ³⁾ | **4** |
| | Glyceryl Stearate (and) PEG-100 Stearate ⁴⁾ | **2** |
| | Behentrimonium Methosulfate (and) Cetyl alcohol (and) Butylene Glycol ⁵⁾ | **4** |
| | Ethoxy (20) Stearyl Alcohol ⁶⁾ | **1** |
| | | |
| **C** | Amodimethicone (and) Trideceth-12 (and) Cetrimonium Chloride ⁷⁾ | **3** |
| | Chlorhexidine Digluconate ⁸⁾ 20% aqueous solution | **0.2** |
| **D** | Citric acid 10% aqueous sol. till pH 3.5-4 | **q.s.** |
| | **TOTAL:** | **100** |

1) Genamin KDM P, Clariant
2) Tylose H10 Y G4, Shin Etsu
3) Lanette O, BASF
4) Arlacel 165-FP-MBAL-PA-(RB), Croda
5) Incroquat Behenyl TMS-50-MBAL-PA-(MH) HA4112, Croda
6) SP Brij S20 MBAL-PA(RB), Croda
7) Xiameter DC MEM-0949 Emulsion, Dow Corning
8) Alfa Aesar

## Claims

1. A process for the preparation of a core-shell microcapsule slurry comprising the steps of:
a) admixing at least one hydrophobic material with at least one polyfunctional 1,1-disubstituted alkene monomer to form an oil phase;
b) adding the oil phase into a water phase comprising a colloidal stabilizer to form an oil-in-water emulsion;
c) applying sufficient conditions to induce interfacial polymerization of the polyfunctional of 1,1-disubstituted alkene monomer so as to form microcapsules in the form of a slurry,
wherein a reactant is added in step a) and/or step b) and or step c).

2. The process according to claim 1 wherein, the polyfunctional 1,1-disubstituted alkene monomer comprises at least one 1,1-disubstituted alkene chosen in the group consisting of methylene malonates, methylene β-ketoesters, methylene β-diketones, dialkyl disubstituted vinyls, dihaloalkyl disustituted vinyls, and mixtures thereof.

3. The process according to claim 1 or 2 wherein, the polyfunctional 1,1-disubstituted alkene monomer comprises at least three methylene malonates covalently bonded.

4. The process according to claim 2 or 3, wherein the polyfunctional monomer is chosen in the group consisting of O',O'-(2-(((2-(ethoxycarbonyl)acryloyl)oxy)methyl)-2-ethylpropane-1,3-diyl) 3-diethyl bis(2-methylenemalonate), O",O',O-(benzene-1,3,5-triyl) 3-triethyl tris(2-methylenemalonate), 2-ethyl-2-(((2-methylene-3-oxobutanoyl)oxy)methyl)propane-1,3-diyl bis(2-methylene-3-oxobutanoate), O',O-(1,4-phenylenebis(methylene)) 3-diethyl bis(2-methylenemalonate), benzene-1,3,5-triyltris(methylene) tris(2-methylene-3-oxobutanoate), benzene-1,3,5-triyltris(methylene) tris(2-methylene-3-oxopentanoate) and mixture thereof.

5. The process according to anyone of the preceding claims, wherein the colloidal stabilizer is chosen in the group consisting of gum Arabic, modified starch, polyvinyl alcohol, polyvinylpyrrolidone, carboxymethylcellulose, anionic polysaccharides, acrylamide copolymer, lignin and derivatives, inorganic particles and mixtures thereof.

6. The process according to anyone of the preceding claims, wherein the reactant is a nucleophile compound chosen in the group consisting of nitrogen nucleophile, sulfur nucleophile, carbon nucleophile, oxygen nucleophile, phosphore nucleophile and mixtures thereof.

7. The process according to anyone of the preceding claims, wherein the reactant is chosen in the group consisting of xylylene diamine, 1,2-diaminocyclohexane, 1,4-diaminocyclohexane, L-lysine, L-Lysine ethyl ester, O,O'-Bis(2-aminopropyl) polypropylene glycol-block-polyethylene glycol-block-polypropylene glycol, ethylene diamine, 1,3-diamino-2-hydroxypropane, diethylene triamine, spermine, spermidine, cystamine, cystine, cystine dialkyl ester, aminoguanidine bicarbonate, N,N'-diethylethylenediamine, polyamidoamine (PAMAM), chitosan, 3-aminopropyltriethoxysilane, L-arginine, 1,3-diaminopropane, N-ethylguanidine sulfate, 1,6-diaminohexane, guanidine salts, N,N,N',N'-tetrakis(3-aminopropyl)-1,4-butanediamine, guanazole, 2-amino-1,3-propanediol, ethanolamine tris(2-aminoethyl)amine, tris(3-aminopropyl)amine, tris[2-(methylamino)ethyl]amine, 1-(2-Aminoethyl)piperazine, triethylenetetramine, triethanolamine, phthalic acid dipotassium salt, succinic acid disodium salt, dithiothreitol, trimethylolpropane tris(3-mercaptopropionate), pentaerythritol tetrakis(3-mercaptopropionate), 2,2'-Thiodiethanethiol and mixtures thereof.

8. The process according to anyone of the preceding claims, wherein the polyfunctional 1,1-disubstituted alkene monomer is added in an amount up to 50% by weight, based on the total weight of the oil phase.

9. The process according to anyone of the preceding claims, wherein the reactant is added in an amount up to 60% by weight, based on the total weight of the oil-in-water dispersion.

10. The process according to anyone of the preceding claims, wherein the hydrophobic active ingredient is a perfume oil.

11. A core-shell microcapsule slurry obtainable by the process as disclosed in anyone of claims 1 to 10.

12. A core-shell microcapsule slurry comprising at least one microcapsule made of:
- a liquid core, preferably an oil based core, and
- a shell comprising at least one polymerized polyfunctional 1,1-disubstituted alkene monomer.

13. A perfuming composition comprising
(i) Perfume microcapsule slurry as defined in claim 11 or 12, wherein the oil-based core comprises a perfume,
(ii) At least one ingredient selected from the group consisting of a perfumery carrier and a perfumery base
(iii) Optionally at least one perfumery adjuvant.

14. A consumer product comprising:
- a personal care active base, and
- microcapsules as defined in claims 11 or 12 or the perfuming composition as defined in claim 13,
wherein the consumer product is in the form of a personal care composition.

15. A consumer product comprising:
- a home care or a fabric care active base, and
- microcapsules as defined in claims 11 or 12 or the perfuming composition as defined in claim 13,
wherein the consumer product is in the form of a home care or a fabric care composition.

## Patentansprüche

1. Verfahren zur Herstellung eines Kern-Hülle-Mikrokapsel-Schlamms, umfassend die Schritte:
a) Mischen mindestens eines hydrophoben Materials mit mindestens einem polyfunktionellen 1,1-disubstituierten Alkenmonomer zwecks Bildung einer Ölphase;
b) Zugeben der Ölphase in eine Wasserphase, umfassend einen kolloidalen Stabilisator, zwecks Bildung einer Öl-in-Wasser-Emulsion;
c) Anwenden ausreichender Bedingungen zur Herbeiführung von Grenzflächenpolymerisation des polyfunktionellen 1,1-disubstituierten Alkenmonomers zwecks Bildung von Mikrokapseln in der Form eines Schlamms,
wobei ein Reaktant in Schritt a) und/oder Schritt b) und/oder Schritt c) zugegeben wird.

2. Verfahren nach Anspruch 1, wobei das polyfunktionelle 1,1-disubstituierte Alkenmonomer mindestens ein 1,1-disubstituiertes Alken umfasst, ausgewählt aus der Gruppe, bestehend aus Methylenmalonaten, Methylen-β-ketoestern, Methylen-β-diketonen, Dialkyl-disubstituierten Vinylen, Dihalogenalkyl-disubstituierten Vinylen und Gemischen davon.

3. Verfahren nach Anspruch 1 oder 2, wobei das polyfunktionelle 1,1-disubstituierte Alkenmonomer mindestens drei kovalent gebundene Methylenmalonate umfasst.

4. Verfahren nach Anspruch 2 oder 3, wobei das polyfunktionelle Monomer ausgewählt ist aus der Gruppe, bestehend aus O',O'-(2-(((2-(Ethoxycarbonyl)acryloyl)oxy)methyl)-2-ethylpropan-1,3-diyl)-3-diethyl-bis(2-methylenmalonat), O",O',O-(Benzen-1,3,5-triyl)-3-triethyl-tris(2-methylenmalonat), 2-Ethyl-2-(((2-methylen-3-oxobutanoyl)oxy)methyl)propan-1,3-diyl-bis(2-methylen-3-oxobutanoat), O',O-(1,4-Phenylenbis(methylen))-3-diethyl-bis(2-methylenmalonat), Benzen-1,3,5-triyltris(methylen)tris(2-methylen-3-oxobutanoat), Benzen-1,3,5-triyltris(methylen)tris(2-methylen-3-oxopentanoat) und Gemischen davon.

5. Verfahren nach einem der vorstehenden Ansprüche, wobei der kolloidale Stabilisator ausgewählt ist aus der Gruppe, bestehend aus Gummiarabikum, modifizierter Stärke, Polyvinylalkohol, Polyvinylpyrrolidon, Carboxymethylcellulose, anionischen Polysacchariden, Acrylamid-Copolymer, Lignin und Derivaten, anorganischen Teilchen und Gemischen davon.

6. Verfahren nach einem der vorstehenden Ansprüche, wobei der Reaktant eine nukleophile Verbindung ist, ausgewählt aus der Gruppe, bestehend aus Stickstoff-Nukleophil, Schwefel-Nukleophil, Kohlenstoff-Nukleophil, Sauerstoff-Nukleophil, Phosphor-Nukleophil und Gemischen davon.

7. Verfahren nach einem der vorstehenden Ansprüche, wobei der Reaktant ausgewählt ist aus der Gruppe, bestehend aus Xylylendiamin, 1,2-Diaminocyclohexan, 1,4-Diaminocyclohexan, L-Lysin, L-Lysinethylester, O,O'-Bis(2-aminopropyl)polypropylenglykol-Block-Polyethylenglykol-Block-Polypropylenglykol, Ethylendiamin, 1,3-Diamino-2-hydroxypropan, Diethylentriamin, Spermin, Spermidin, Cystamin, Cystin, Cystindialkylester, Aminoguanidinbicarbonat, N,N'-Diethylethylendiamin, Polyamidoamin (PAMAM), Chitosan, 3-Aminopropyltriethoxysilan, L-Arginin, 1,3-Diaminopropan, N-Ethylguanidinsulfat, 1,6-Diaminohexan, Guanidinsalzen, N,N,N',N'-Tetrakis(3-aminopropyl)-1,4-butandiamin, Guanazol, 2-Amino-1,3-propandiol, Ethanolamin, Tris(2-aminoethyl)amin, Tris(3-aminopropyl)amin, Tris[2-(methylamino)ethyl]amin, 1-(2-Aminoethyl)piperazin, Triethylentetramin, Triethanolamin, Phthalsäure Dikaliumsalz, Bernsteinsäure Dinatriumsalz, Dithiothreitol, Trimethylolpropan-tris(3-mercaptopropionat), Pentaerythritol-tetrakis(3-mercaptopropionat), 2,2'-Thiodiethanthiol und Gemischen davon.

8. Verfahren nach einem der vorstehenden Ansprüche, wobei das polyfunktionelle 1,1-disubstituierte Alkenmonomer in einer Menge von bis zu 50 Gew.-%, bezogen auf das Gesamtgewicht der Ölphase, zugegeben wird.

9. Verfahren nach einem der vorstehenden Ansprüche, wobei der Reaktant in einer Menge von bis zu 60 Gew.-%, bezogen auf das Gesamtgewicht der Öl-in-Wasser-Dispersion, zugegeben wird.

10. Verfahren nach einem der vorstehenden Ansprüche, wobei der hydrophobe aktive Inhaltsstoff ein Parfümöl ist.

11. Kern-Hülle-Mikrokapsel-Schlamm, erhaltbar durch das Verfahren, wie es in einem der Ansprüche 1 bis 10 offenbart ist.

12. Kern-Hülle-Mikrokapsel-Schlamm, umfassend mindestens eine Mikrokapsel, hergestellt aus:
- einem flüssigen Kern, vorzugsweise einem Kern auf Ölbasis, und
- einer Hülle, umfassend mindestens ein polymerisiertes polyfunktionelles 1,1-disubstituiertes Alkenmonomer.

13. Parfümierende Zusammensetzung, umfassend
(i) Parfüm-Mikrokapsel-Schlamm gemäß Definition in Anspruch 11 oder 12, wobei der Kern auf Ölbasis ein Parfüm umfasst,
(ii) mindestens eine Ingredienz, ausgewählt aus der Gruppe, bestehend aus einem Parfümträger und einer Parfümbasis,
(iii) wahlweise mindestens einen Parfümhilfsstoff.

14. Verbraucherprodukt, umfassend:
- eine aktive Körperpflegebasis, und
- Mikrokapseln gemäß Definition in Ansprüchen 11 oder 12 oder die parfümierende Zusammensetzung gemäß Definition in Anspruch 13,
wobei das Verbraucherprodukt in der Form einer Körperpflegezusammensetzung vorliegt.

15. Verbraucherprodukt, umfassend:
- eine aktive Haushaltspflege- oder Textilpflegebasis, und
- Mikrokapseln gemäß Definition in Ansprüchen 11 oder 12 oder die parfümierende Zusammensetzung gemäß Definition in Anspruch 13,
wobei das Verbraucherprodukt in der Form einer Haushaltspflege- oder einer Textilpflegezusammensetzung vorliegt.

## Revendications

1. Procédé de préparation d'une suspension épaisse de microcapsules à noyau-enveloppe, comprenant les étapes consistant à :
a) mélanger au moins un matériau hydrophobe avec au moins un monomère alcène 1,1-disubstitué polyfonctionnel pour former une phase huileuse ;
b) ajouter la phase huileuse dans une phase aqueuse comprenant un stabilisant colloïdal pour former une émulsions huile-dans-eau ;
c) appliquer des conditions suffisantes pour induire une polymérisation interfaciale du monomère alcène 1,1-disubstitué polyfonctionnel de façon à former des microcapsules sous la forme d'une suspension épaisse,
dans lequel un réactif est ajouté à l'étape a) et/ou l'étape b) et/ou l'étape c).

2. Procédé selon la revendication 1 dans lequel le monomère alcène 1,1-disubstitué polyfonctionnel comprend au moins un alcène 1,1-disubstitué choisi dans le groupe constitué par les méthylènemalonates, les méthylène-β-cétoesters, les méthylène-β-dicétones, les dialkylvinyles disubstitués, les dihalogénoalkylvinyles disubstitués, et des mélanges de ceux-ci.

3. Procédé selon la revendication 1 ou 2 dans lequel le monomère alcène 1,1-disubstitué polyfonctionnel comprend au moins trois méthylènemalonates liés par covalence.

4. Procédé selon la revendication 2 ou 3 dans lequel le monomère polyfonctionnel est choisi dans le groupe constitué par le O',O'-(2-(((2-(éthoxycarbonyl)acryloyl)oxy)méthyl)-2-éthylpropane-1,3-diyl)-3-diéthyl-bis(2-méthylènemalonate), le O",O',O-(benzène-1,3,5-triyl)-3-triéthyl-tris(2-méthylènemalonate), le 2-éthyl-2-(((2-méthylène-3-oxobutanoyl)oxy)méthyl)propane-1,3-diyl-bis(2-méthylène-3-oxobutanoate), le O',O-(1,-phénylènebis(méthylène))-3-diéthyl-bis(2-méthylènemalonate), le benzène-1,3,5-triyltris(méthylène)tris(2-méthylène-3-oxobutanoate), le benzène-1,3,5-triyltris(méthylène)tris(2-méthylène-3-oxopentanoate) et des mélanges de ceux-ci.

5. Procédé selon l'une quelconque des revendications précédentes, dans lequel le stabilisant colloïdal est choisi dans le groupe constitué par la gomme arabique, l'amidon modifié, l'alcool polyvinylique, la polyvinylpyrrolidone, la carboxyméthylcellulose, les polysaccharides anioniques, un copolymère d'acrylamide, la lignine et des dérivés de celle-ci, des particules inorganiques, et des mélanges de ceux-ci.

6. Procédé selon l'une quelconque des revendications précédentes, dans lequel le réactif est un composé nucléophile choisi dans le groupe constitué par un nucléophile azoté, un nucléophile soufré, un nucléophile carboné, un nucléophile oxygéné, un nucléophile phosphoré, et des mélanges de ceux-ci.

7. Procédé selon l'une quelconque des revendications précédentes, dans lequel le réactif est choisi dans le groupe constitué par la xylylènediamine, le 1,2-diaminocyclohexane, le 1,4-diaminocyclohexane, la L-lysine, l'ester éthylique de L-lysine, le O,O'-bis(2-aminopropyl)polypropylèneglycol-bloc-polyéthylèneglycol-bloc-polypropylèneglycol, l'éthylènediamine, le 1,3-diamino-2-hydroxypropane, la diéthylènetriamine, la spermine, la spermidine, la cystamine, la cystine, un ester dialkylique de cystine, le bicarbonate d'aminoguanidine, la N,N'-diéthyléthylènediamine, la polyamidoamine (PAMAM), le chitosane, le 3-aminopropyltriéthoxysilane, la L-arginine, le 1,3-diaminopropane, le sulfate de N-éthylguanidine, le 1,6-diaminohexane, les sels de guanidine, la N,N,N',N'-tétrakis(3-aminopropyl)-1,4-butanediamine, le guanazole, le 2-amino-1,3-propanediol, l'éthanolamine, la tris(2-aminoéthyl)amine, la tris(3-aminopropyl)amine, la tris[2-(méthylamino)éthyl]amine, la 1-(2-aminoéthyl)pipérazine, la triéthylènetétramine, la triéthanolamine, le sel dipotassique de l'acide phtalique, le sel disodique de l'acide succinique, le dithiothréitol, le tris(3-mercaptopropionate) de triméthylolpropane, le tétrakis(3-mercaptopropionate) de pentaérythritol, le 2,2'-thiodiéthanethiol et des mélanges de ceux-ci.

8. Procédé selon l'une quelconque des revendications précédentes, dans lequel le monomère alcène 1,1-disubstitué polyfonctionnel est ajouté en une quantité allant jusqu'à 50% en poids, par rapport au poids total de la phase huileuse.

9. Procédé selon l'une quelconque des revendications précédentes, dans lequel le réactif est ajouté en une quantité allant jusqu'à 60% en poids, par rapport au poids total de la dispersion huile-dans-eau.

10. Procédé selon l'une quelconque des revendications précédentes, dans lequel l'ingrédient actif hydrophobe est une huile de parfum.

11. Suspension épaisse de microcapsules à noyau-enveloppe, pouvant être obtenue par le procédé tel que décrit dans l'une quelconque des revendications 1 à 10.

12. Suspension épaisse de microcapsules à noyau-enveloppe, comprenant au moins une microcapsule constituée de :
- un noyau liquide, de préférence un noyau à base d'huile, et
- une enveloppe comprenant au moins un monomère alcène 1,1-disubstitué polyfonctionnel polymérisé.

13. Composition parfumante comprenant
(i) une suspension épaisse de microcapsules de parfum telle que définie dans la revendication 11 ou 12, dans laquelle le noyau à base d'huile comprend un parfum,
(ii) au moins un ingrédient sélectionné dans le groupe constitué par un support de parfumerie et une base de parfumerie
(iii) facultativement, au moins un adjuvant d'usage courant en parfumerie.

14. Produit de consommation comprenant :
- une base active pour soins corporels, et
- des microcapsules telles que définies dans les revendications 11 ou 12 ou la composition parfumante telle que définie dans la revendication 13,
où le produit de consommation est sous la forme d'une composition de soins corporels.

15. Produit de consommation comprenant:
- une base active pour l'entretien ménager ou l'entretien textile, et
- des microcapsules telles que définies dans les revendications 11 ou 12 ou la composition parfumante telle que définie dans la revendication 13,
où le produit de consommation est sous la forme d'une composition pour l'entretien ménager ou l'entretien textile.
